(19) Europäisches Patentamt
European Patent Office
Office européen des brevets

(11) Veröffentlichungsnummer: **0 003 338**
**B1**

(12) **EUROPÄISCHE PATENTSCHRIFT**

(45) Veröffentlichungstag der Patentschrift: **09.03.83**

(51) Int. Cl.³: **C 08 K 5/13**, C 08 L 21/02 //C07C39/12, C07C39/17

(21) Anmeldenummer: **79100165.4**

(22) Anmeldetag: **19.01.79**

(54) **Verwendung von Benzylphenolen als Antioxydantien.**

(30) Priorität: **01.02.78 DE 2804215**

(43) Veröffentlichungstag der Anmeldung:
**08.08.79 Patentblatt 79/16**

(45) Bekanntmachung des Hinweises auf die Patenterteilung:
**09.03.83 Patentblatt 83/10**

(84) Benannte Vertragsstaaten:
**BE CH DE FR GB IT NL**

(56) Entgegenhaltungen:
**FR - A - 1 300 990**
**FR - A - 1 410 914**
**GB - A - 806 014**
**GB - A - 885 005**
**GB - A - 1 018 318**
**US - A - 3 875 246**

**CHEMICAL ABSTRACTS, Vol. 70, Nr. 17, 28-04-1969, Zusammenfassung Nr. 78708b, Columbus, Ohio, USA, MINSKER k:S. et al.: "Half-wave potentials for the electrical oxidation of substituted phenols and the effectiveness of their action as poly(vinyl chloride) stabilizers". Seite 31, rechte Spalte**

(73) Patentinhaber: **BAYER AG**
**Zentralbereich Patente, Marken und Lizenzen**
**D-5090 Leverkusen 1, Bayerwerk (DE)**

(72) Erfinder: **Buysch, Hans-Josef, Dr.**
**Brandenburger Strasse 28**
**D-4150 Krefeld 1 (DE)**
Erfinder: **Matner, Martin, Dr.**
**Dorfstrasse 14**
**D-5068 Odenthal (DE)**
Erfinder: **Freese, Hans, Dr.**
**Pastor-Scheibler-Strasse 22**
**D-5090 Leverkusen 31 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 003 338 B1

Courier Press, Leamington Spa, England.

Verwendung von Benzylphenolen als Antioxidantien

Die vorliegende Erfindung betrifft die Verwendung von Benzylphenolen der allgemeinen Formel I

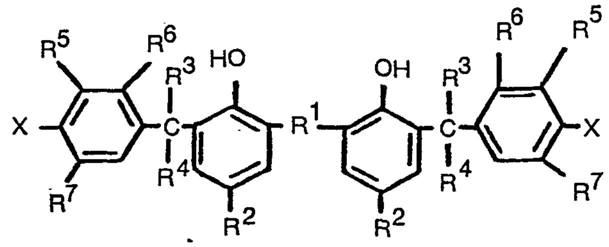

(I)

in der $R^1$ einer der folgenden zweiwertigen Reste

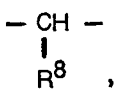,
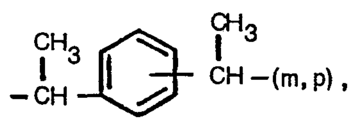,
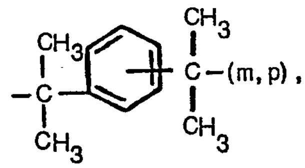,
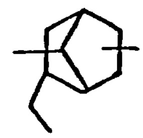,

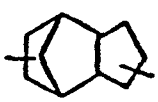,
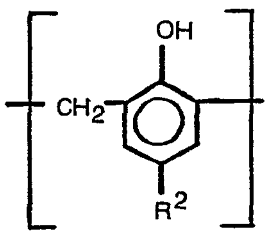 1-5,
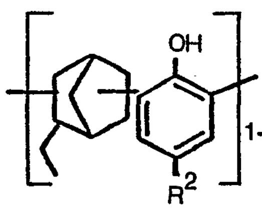 1-5,
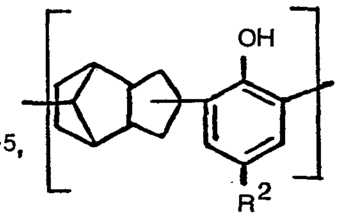 1-5,

$R^2$ = $C_1$—$C_9$-Alkyl, Benzyl, $\alpha$-Methylbenzyl, $\alpha,\alpha$-Dimethylbenzyl
$R^3$ = H, $CH_3$
$R^4$ = H, $CH_3$
$R^5$ = H, $C_1$—$C_4$-Alkyl
$R^6$ = H
$R^7$ = H, $C_1$—$C_4$-Alkyl
$R^8$ = H, $C_1$—$C_3$-Alkyl und
X = H, $C_1$—$C_3$-Alkyl ist,
zur Stabilisierung von Kautschuk oder Kautschuk-Latices.

Besonders bevorzugt sind

$R^2$ = $CH_3$
$R^5$ = H, tert.-Butyl
$R^7$ = H, tert.-Butyl, Isopropyl
X = H, Isopropyl.

Genannt seien beispielsweise von den erfindungsgemäßen Benzylphenolen Bis-(2-hydroxy-3-benzyl-5-methyl-phenyl)-methan sowie dessen höhermolekulare Homologe, in denen

$R^1$ = 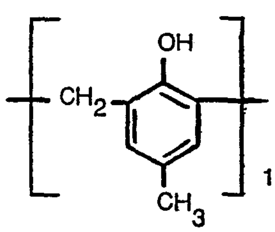 1–5 ist,

Bis-[2-hydroxy-3-($\alpha$-cumyl)-5-methyl-phenyl]-methan,
Bis-[2-hydroxy-3-($\alpha$-methylbenzyl)-5-methyl-phenyl]-methan,
Bis-(2-hydroxy-3,5-dibenzyl-phenyl)-methan,

1,1-Bis-(2-hydroxy-3-benzyl-5-methyl-phenyl)-isobutan,
Bis-(2-hydroxy-3-benzyl-5-tert.-butyl-phenyl)-methan,
1,1-Bis-(2-hydroxy-3-benzyl-5-methyl-phenyl)-ethan,
Bis-(2-hydroxy-3-benzyl-5-methyl-phenyl)-2-ethylnorbornan
und höhermolekulare Homologe mit

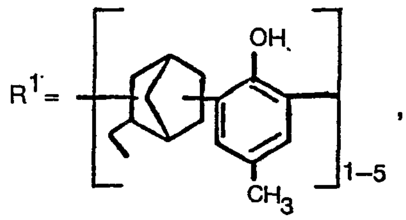

Bis-(2-hydroxy-3-benzyl-5-methyl-phenyl)-tricyclo-(5.2.1.$0^{2.6}$)-decan und höhermolekulare Homologe mit

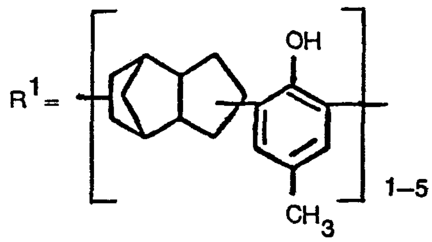

$\alpha,\alpha'$-Bis-(2-hydroxy-3-benzyl-5-methyl-phenyl)-m- und -p-diethylbenzol,
Bis-(2-hydroxy-3-benzyl-5-isononyl-phenyl)-methan,
Bis-[2-hydroxy-3-($\alpha,\alpha$-dimethyl-p-isopropyl-benzyl)-5-methyl-phenyl]-methan,
Bis-[2-hydroxy-3-($\alpha,\alpha$-p-trimethyl-benzyl)-5-methyl-phenyl]-methan,
Bis-[2-hydroxy-3-($\alpha,\alpha$-dimethyl-p-hydroxy-benzyl)-5-methyl-phenyl]-methan,
Bis-[2-hydroxy-3,5-di-($\alpha$-cumyl)-phenyl]-methan,
Bis-[2-hydroxy-3,5-di-($\alpha$-styrol)-phenyl]-methan.

Es wird durch die vorliegende Anmeldung die Lehre gegeben, daß diese Benzylphenole zur Stabilisierung von Kautschuk geeignet sind. Dies war auch im Hinblick auf die FR—A 1 300 990 nicht vorhersehbar. Die dort beschriebenen Verbindungen unterscheiden sich strukturell ganz wesentlich durch zusätzliche Hydroxylgruppen in den Benzylresten. Die Angaben auf Seite 1, Spalte 1 und Seite 5, Spalte 1 über die Stabilisierung von z.B. Schmierölen und Kautschuk gegen oxidativen Abbau beziehen sich nicht allgemein auf mehrkernige phenolische Antioxidantien, sondern betreffen lediglich die dort beschriebenen speziellen Verbindungen. Aus der FR—PS 1 300 990 konnte daher die Verwendung von Benzylphenolen entsprechend der vorliegenden Erfindung nicht nahegelegt werden.

Die aus der GB—A 806 014 bekannten Stabilisatoren für Kautschuk weisen als spezifisches Strukturelement die Verknüpfung der Phenolgruppen über einen Xylolrest auf. Es gibt keinen Hinweis, daß für Kautshuk-Stabilisatoren beliebige andere Brückenelemente geeignet sein könnten. Es hat daher nicht auf der Hand gelegen, z.B. eine Verbindung gemäß Beispiel 10 für diesen Zweck einzusetzen, bei der die Phenolgruppen über einen durch verzweigte Alkylgruppen substituierten Phenylrest verknüpft sind.

Die erfindungsgemäß verwendeten Verbindungen sind grundsätzlich auf zwei Wegen zugänglich:

1) Man verknüpft zuerst Phenole der Formel II

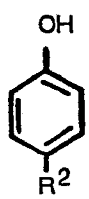

(II)

worin $R^2$ die oben genannte Bedeutung hat, mit den Rest $R^1$ und entsprechende Funktionen enthaltenden Verbindungen, so daß Substanzen der Formel III

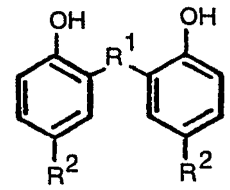

(III)

entstehen, worin

$R^1$ und $R^2$ die oben genannte Bedeutung besitzen und kondensiert die Substanzen III mit Benzylverbindungen der Formel IV bzw. daraus abgeleiteten Olefinen der Formel IVa

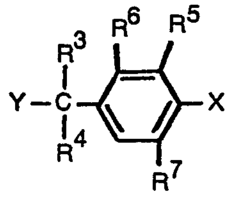

(IV)

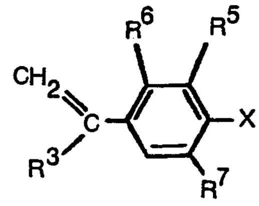

(IVa)

worin Y = Halogen, OH, O-Alkyl, O-Acyl sein kann, unter Bildung der erfindungsgemäßen Benzylphenole I.

2) In diesem Fall verfährt man umgekehrt, indem man zunächst Benzylphenole der Formel V

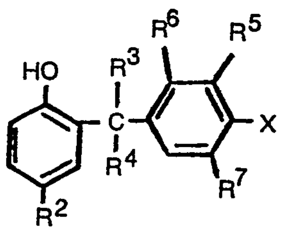

(V)

synthetisiert durch Kondensation von Phenolen der Formel II mit Benzylverbindungen IV bzw. IVa und dann die Verknüpfung der Benzylphenole V durch den Rest $R^1$ vornimmt. Geeignete Ausgangsprodukte für die Herstellung der erfindungsgemäßen Benzylphenole I sind einerseits p-substituierte Phenole II wie z.B. p-Kresol, p-ethylphenol, p-Isopropylphenol, p-tert.-Butylphenol, p-tert.-Amylphenol, p-Cyclohexylphenol, p-Benzylphenol, p-Styrylphenol und p-Cumylphenol und andererseits Benzylverbindungen IV bzw. IVa wie beispielsweise Benzylchlorid, Benzylalkohol, o-, m- und p-Methyl-benzylchlorid, o-, m- und p-Hydroxy-benzylalkohol, 3,5-Dimethyl-4-hydroxybenzylalkohol, 3,5-Di-tert-butyl-4-hydroxybenzylacetat, Styrol, p-Methylstyrol, $\alpha$-Methylstyrol, p-Hydroxy-styrol, p-Isopropenylphenol, p-Methyl-$\alpha$-methyl-styrol, m- und p-Isopropyl-$\alpha,\alpha$-dimethylbenzylalkohol und m- und p-Isopropyl-$\alpha$-methylstyrol.

Geeignete Ausgangsprodukte für die Ausbildung des Brückengliedes $R^1$ sind bifunktionelle Alkylierungsmittel wie 1,2-, 1,3- oder 1,4-Bis-chlormethylbenzol, 1,3- und 1,4-$\alpha,\alpha$-Dihydroxy-diisopropylbenzol, Divinylbenzol, 1,4-Cyclohexandimethanol, 5-Ethyliden-norbornen, Vinylcyclohexan, Dicyclopentadien, 1,4-Dihydroxycyclohexan und Cyclopentadien, ferner solche Verbindungen, die bei den obenerwähnten Syntheseschritten als bifunktionelle Alkylierungsmittel reagieren, wie z.B. Aceton, Formaldehyd, Acetaldehyd, Propionaldehyd, Butyr- und Isobutyraldehyd, Cyclopentyl-aldehyd, Cyclohexylaldehyd, Cyclohexenylaldehyd und Önanthaldehyd.

Für den Fall, daß $R^1$ eine Einfachbindung darstellt, werden die p-Alkylphenole II nach bekannten Methoden oxydativ verknüpft, wie sie z.B. in Org. Chem. 1063 (1963) beschrieben sind, und dann mit den Benzylverbindungen IV oder IVa umgesetzt.

Alle Syntheseschritte zur Darstellung der erfindungsgemäßen Benzylphenole werden nach bekannten Verfahren durchgeführt, die z.B. beschrieben sind in Houben-Weyl, "Methoden der Organischen Chemie", Band 6/1c, S. 925 ff, 955 ff, 993 ff, 1002 ff, 1021 ff.

Die erfindungsgemäßen Verbindungen sind ausgezeichnete Stabilisatoren für Doppelbindungen enthaltende Polymerisate wie z.B. Naturkautschuk, Styrol-Butadien-Kautschuk, (SBR), Nitrilkautschuk und Chloroprenkautschuk. Besondere Wirkung besitzen diese Substanzen bei der Stabilisierung von Kautschuklatices, insbesondere von SBR-Latices. Kautschukmaterialien, die damit stabilisiert sind, besitzen gegenüber der aus dem Stand der Technik bekannten Stabilisierung mit phenolischen Antioxydantien eine erheblich verbesserte Beständigkeit bei thermooxydativer Belastung und weit geringere

Verfärbung im Licht. Außerdem können diese günstigen Effekte schon mit einem Bruchteil der Menge an Stabilisatoren erreicht werden, die üblicherweise (ca. 1—2 Gew.-%) in den Kautschuk eingearbeitet werden.

Die folgenden Beispiele erläutern die Erfindung. Die Prozentangaben sind stets Gewichtsprozent.

Beispiel 1

*Bis-(2-hydroxy-3-benzyl-5-methyl-phenyl)-methan*

Ein Gemisch von 396 g (2,0 Mol) o-Benzyl-p-kresol, 86 g (1 Mol) 35%iges Formalin und 8 g Oxalsäure wird 22 h unter $N_2$, Rühren und Rückfluß erhitzt. Anschließend werden flüchtige Bestandteile und wenig umgesetztes o-Benzyl-p-kresol bis zu einer Sumpftemperatur von 210°C/19 Torr abdestilliert. Man erhält als Sumpfprodukt ein gelbliches Weichharz (406 g) mit einem Gehalt an phenolischen OH-Gruppen von 8,25% (ber. 8,23%).

*1a) o-Benzyl-p-kresol*

Ein Gemisch von 864 g (8,0 Mol) p-Kresol und 252 g (2,0 Mol) Benzylchlorid wird solange auf 150—180°C erhitzt, bis kein HCl-Gas entweicht. Das Reaktionsprodukt wird fraktioniert destilliert, wobei zunächst nicht umgesetztes Ausgangsprodukt übergeht, dann bei 130—135°C/0,6 Torr o-Benzyl-p-kresol.

o-Benzyl-p-kresol kann jedoch auch ohne destillative Reinigung als Rohprodukt eingesetzt werden.

1b) Eine andere Möglichkeit zur Herstellung von o-Benzyl-p-kresol besteht in der Kondensation von Benzylalkohol mit p-Kresol in Gegenwart saurer Katalysatoren:

Ein Gemisch von 432 g (4 Mol) p-Kresol, 108 g (1 Mol) Benzylalkohol und 0,5 g p-Toluolsulfonsäure wird unter $N_2$ und Rühren in 2—3 h auf ca. 145—165°C erhitzt, wobei das Reaktionswasser übergeht und sich im Abscheider sammelt.

Anschließend erhitzt man ca. 1 h auf 185—198°C. Nach Abdestillieren von überschüssigem Kresol erhält man ein gelbes bis hellbraunes Öl, das unmittelbar mit Formalin kondensiert werden kann.

Das Produkt kann auch destillativ gereinigt werden. Man erhält aus dem obigen Ansatz

| | | | |
|---|---|---|---|
| 153 g | Monobenzylkresol | $n_D^{20}$ | 1,5916 |
| 31 g | Dibenzylkresol | " | 1,609 |
| 5 g | Rückstand | | |

Beispiel 2

*Bis-(2-hydroxy-3-benzyl-5-methyl-phenyl)-methan*

Analog Beispiel 1: Statt des reinen o-Benzylkresols wird das Rohprodukt der Umsetzung von p-Kresol mit Benzylchlorid aus 1a eingesetzt, das man als Sumpfprodukt erhält, wenn man nach Aufhören der HCl-Entwicklung lediglich nicht umgesetztes Ausgangsprodukt bis zu einer Sumpftemperatur von 170—175°C bei 17 Torr abdestilliert. Als Reaktionsprodukt bleibt man ein Gemisch zurück, das neben der Hauptkomponente Bis-(2-hydroxy-3-benzyl-5-methyl-phenyl)-methan noch Dibenzyl-p-kresol enthält.

Beispiel 3

*Bis-(2-hydroxy-3-benzyl-5-methyl-phenyl)-methan*

Ein Gemisch von 228 g (1 Mol) Bis-(2-hydroxy-5-methyl-phenyl)-methan, 216 g (2 Mol) Benzylalkohol, 1 g Toluolsulfonsäure und 100 g Xylol als Schleppmittel wird auf 140—160°C Sumpftemperatur erhitzt, wobei Wasser azeotrop übergeht.

Nachdem 36 g Wasser übergegangen sind, wird mit Sodalösung neutralisiert. Dann werden im Vakuum bis 170°C/12 Torr flüchtige Anteile abdestilliert. Als Rückstand erhält man ein gelbbraunes Harz. Falls erforderlich, kann das Na-Toluolsulfonat durch Wasser in der Hitze ausgewaschen werden.

Beispiel 4

*Bis-(2-hydroxy-3-benzyl-5-methyl-phenyl)-methan*

Setzt man analog Beispiel 3 ein Gemisch aus etwa 60 Gew.-% Bis-(2-hydroxy-5-methyl-phenyl)-methan und 40 Gew.-% seiner höheren Homologen (Novolake) ein, wie man sie im allgemeinen bei der Kondensation von p-Kresol mit Formalin erhält, und kondensiert mit Benzylalkohol, so gewinnt man ein gelbes Harz, das außer der Titelverbindung noch höhermolekulare Produkte der idealisierten Struktur

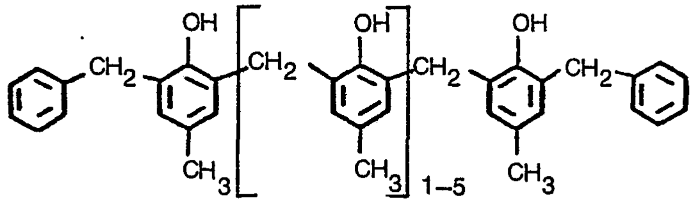

enthält.

Beispiel 5

*Bis-(2-hydroxy-3-(α-*methyl-benzyl)-5-methyl-phenyl)-methan

Ein Gemisch von 257 g (1,21 Mol) o-Styryl-p-kresol, 52 g (0,6 Mol) 35%iges Formalin und 7 g konz. Salzsäure wird 6 h unter $N_2$, Rühren und Rückfluß erhitzt. Nach Abdestillieren flüchtiger Bestandteile bis zu einer Sumpftemperatur von 200°C/12 Torr bleiben 268 g eines gelbbraunen Harzes zurück mit einem Gehalt an phenolischer OH-Gruppen von 7,45% (ber. 7,57%).

Beispiel 6

*Bis-(2-hydroxy-3,5-dibenzyl-phenyl)-methan*

Ein Gemisch von 274 g (1,0 Mol) 2,4-Dibenzylphenol, 43 g (0,5 Mol) 35%iges Formalin und 4 g Oxalsäure wird 22 h unter $N_2$, Rühren und Rückfluß erhitzt. Nach Abtreiben flüchtiger Bestandteile bis zu einer Sumpftemperatur von 260°C/0,7 Torr bleibt 98 g hellbraunes Harz als Rückstand mit einem Gehalt an phenolischen OH-Gruppen von 5,9% (ber. 6,07%).

Beispiel 7

*1,1-Bis-(2-hydroxy-3-benzyl-5-methyl-phenyl)-isobutan*

396 g (2 Mol) o-Benzyl-p-kresol werden bei Raumtemperatur mit HCl-Gas gesättigt. Dazu tropft man bei 40—50°C eine Lösung von 72 g (1 Mol) Isobutyraldehyd in 396 g (2 Mol) o-Benzyl-p-kresol unter Rühren und HCl-Einleiten in 2 h zu, läßt über Nacht stehen und destilliert. Nach einem Vorlauf von Kondensationswasser und Benzylkresol geht das Reaktionsprodukt bei 227—235°/0,2 Torr als zähflüssiges Harz über. Phenolische OH-Gruppen: Gef. 7,20%, Ber. 7,38%.

Beispiel 8

*Bis-(2-hydroxy-3-benzyl-5-tert.-butyl-phenyl)-methan*

Ein Gemisch von 120 g (0,5 Mol) p-tert.-Butyl-o-benzylphenol, 21,5 g (0,25 Mol) 35%iges Formalin und 2 g Oxalsäure wird 13 h unter $N_2$ und Rückfluß gekocht.

Nach Abtreiben flüchtiger Bestandteile bis 200°C Sumpftemperatur und 10 Torr bleiben 120 g eines hellbraunen Weichharzes als Reaktionsprodukt zurück.

Beispiel 9

*1,1-Bis-(2-hydroxy-3-benzyl-5-methyl-phenyl)-ethan*

Analog Beispiel 11 wird Acetaldehyd mit Benzylkresol kondensiert. Man erhält ein Weichharz, das bei 218—225°C/0,2 Torr übergeht.

Beispiel 10

*Bis-(2-hydroxy-3-benzyl-5-methyl-phenyl)-Ethylnorbornan*

Ein Gemisch von 540 g (5 Mol) p-Kresol und 20 g $BF_3$-Hydrat wird unter Stickstoff und Rühren bei 70—80°C in 2—3 h tropfenweise mit 300 g (2,5 Mol) Ethylennorbornen versetzt und dann noch 1 h bei ca. 100°C gehalten.

Das Reaktionsgemisch wird mit 400 ml Ligroin verdünnt, mit Sodalösung und Wasser neutral gewaschen, mit Natriumsulfat getrocknet und filtriert.

Nach Zugabe von 2,0 g Zinkchlorid tropft man bei 100—110°C 570 g (4,5 Mol) Benzylchlorid unter HCl-Entwicklung in 6—7 h zu und heizt langsam höher bis nach weiteren 8 h eine Sumpftemperatur von 130—135°C erreicht und die Gasentwicklung beendet ist. Das Reaktionsprodukt wird mit Ligroin versetzt, mit verdünnter Salzsäure und mehrmals mit Wasser gewaschen, bis das Waschwasser neutral reagiert und schließlich im Wasserstrahlvakuum von flüchtigen Substanzen befreit. Man erhält ein braunes Harz mit einem Gehalt an phenolischen OH-Gruppen von 2,3%. Es enthält nicht nur die Titelverbindung, sondern neben Benzylkresolen auch höhermolekulare Anteile der idealisierten Struktur

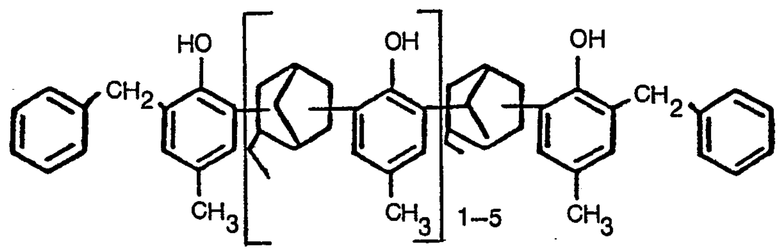

Der Anteil an Benzylkresolen läßt sich vermeiden, wenn man das primäre Reaktionsgemisch nach der Umsetzung von Kresolen mit Ethylennorbornen destillativ von nicht umgesetztem p-Kresol befreit.

Beispiel 11

*Bis-(2-hydroxy-3-benzyl-5-methyl-phenyl)-tricyclo-(5.2.1.0$^{2.6}$)-decan*

Ein Gemisch von 540 g (5 Mol) p-Kresol und 20 g $BF_3$-Hydrat wird unter Rühren und Stickstoff in 2—3 h bei 70—90°C mit 330 g (2,5 Mol) Dicyclopentadien versetzt und noch 2 h bei 90°C gehalten. Anschließend tropft man 2—3 h bei 120—130°C 485 g (4,5 Mol) Benzylalkohol hinzu, wobei Wasser abgespalten wird. Man heizt nun nach und nach auf 140—150°C, so daß nach weiteren 2—3 h kein Wasser mehr abdestilliert. Das Reaktions-gemisch wird mit Toluol verdünnt, mit Sodalösung und Wasser neutral gewaschen, getrocknet und wieder vom Toluol befreit. Man erhält ein braunes Harz, das neben der Titelverbindung noch Mono- und Dibenzylkresol und höhermolekulare Verbindungen der idealisierten Struktur

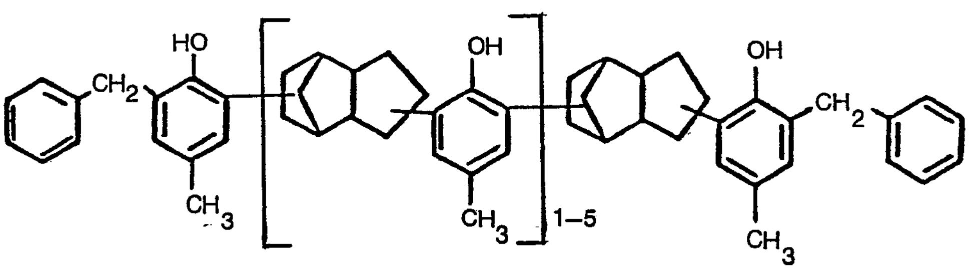

enthält.

Mono- und Dibenzylkresol treten nicht auf, wenn man nach der Umsetzung mit Dicyclopentadien das Reaktionsgemisch destillativ von Überschüssigem p-Kresol befreit.

Die Prüfung der Produkte erfolgte nach folgendem Verfahren:

Eine Kautschukdispersion, hergestellt durch Emulsionspolymerisation von 55% Styrol, 41% Butadien, 2% Acrylsäure und 2% Methacrylsäure, wird mit 0,25 Gewichtsteilen (bezogen auf 100 g Kautschuk) der erfindungsgemäßen Produkte versetzt. Die Zugabe erfolgt in Form einer 30%igen bzw. 50%igen Emulsion in Wasser. Nach einer Standzeit von ca. 1 Tag wird durch Antrocknen bei Raumtemperatur ein Kautschukfilm hergestellt. Dieser wird bei 110°C 15 Minuten lang kondensiert. Nach der DIN-Vorschrift 53504 werden aus dem so behandelten Film Prüfkörper (S III-Stäbe) hergestellt.

Die Alterung der Proben erfolgt in einem Heißluftschrank bei 140°C über einen Zeitraum von 6, 24, 48 und 72 Stunden. Die folgende Bestimmung der Bruchdehnung wird wiederum nach DIN 53504 vorgenommen, wobei der zunehmende Abfall der Bruchdehnung nach Alterung als Maß für das Alterungsverhalten (Verhärtung) dient.

Für die in den Beispielen 2, 5 und 6 beschriebenen Produkte wurden folgende Ergebnisse erzielt. Als Vergleich dient der nicht stabilisierte Kautschukfilm und ein mit einem handelsüblichen Alterungsschutzmittel (Bis-(2-hydroxy-3-iso-nonyl-5-methyl-phenyl)-methan) stabilisierter Film.

| Alterungsschutzmittel | Bruchdehnung nach 72 Stunden Alterung bei 140°C (%) |
|---|---|
| — | 0 |
| Bis-(2-hydroxy-3-iso-nonyl-5-methyl-phenyl)-methan | 200 |
| Beispiel 2 | 230 |
| Beispiel 5 | 420 |
| Beispiel 6 | 220 |

**Patentanspruch**

Verwendung von Benzylphenolen der allgemeinen Formel I

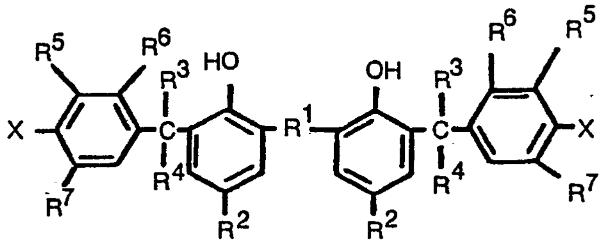

(I)

in der $R^1$ einer der folgenden zweiwertigen Reste

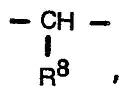 , 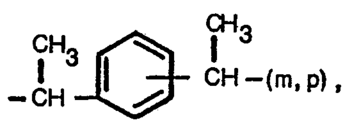 , 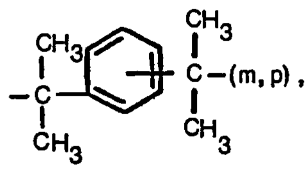 , 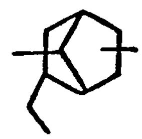 ,

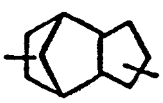 , 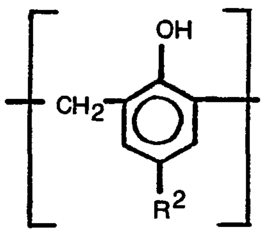 1-5, 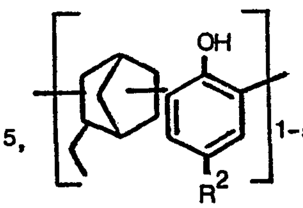 1-5, 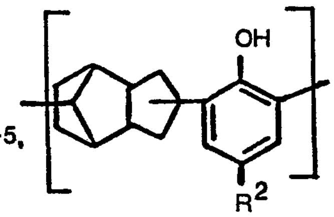 1-5,

$R^2$ = $C_1$—$C_9$-Alkyl, Benzyl, $\alpha$-Methylbenzyl, $\alpha,\alpha$-Dimethylbenzyl
$R^3$ = H, $CH_3$
$R^4$ = H, $CH_3$
$R^5$ = H, $C_1$—$C_4$-Alkyl
$R^6$ = H
$R^7$ = H, $C_1$—$C_4$-Alkyl
$R^8$ = H, $C_1$—$C_3$-Alkyl und
X = H, $C_1$—$C_3$-Alkyl ist,
zur Stabilisierung von Kautschuk oder Kautschuk-Latices.

**Claim**

Use of benzyl phenols of the general formula I

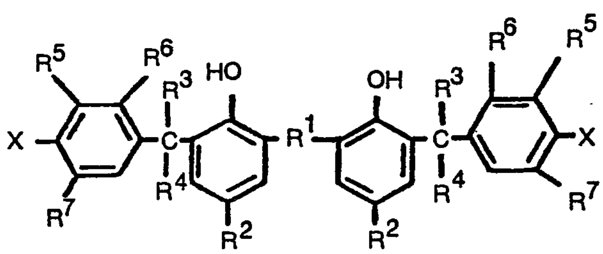

(I)

in which

$R^1$ is one of the following difunctional radicals

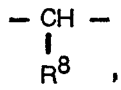 , 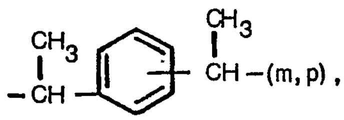 , 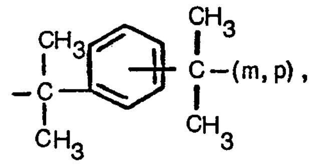 , 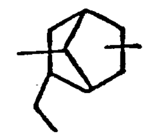 ,

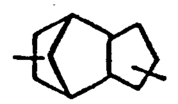 , 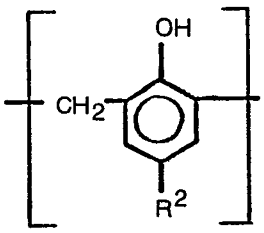 1-5, 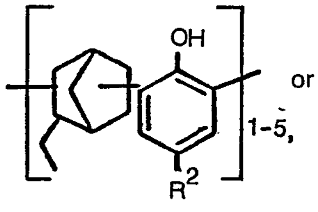 1-5, or 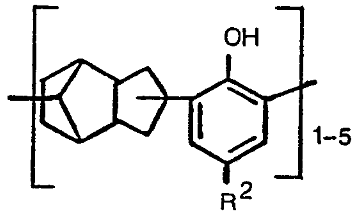 1-5,

$R^2$ = $C_1$—$C_9$ alkyl, benzyl, $\alpha$-methyl benzyl or $\alpha,\alpha$-dimethyl benzyl
$R^3$ = H or $CH_3$
$R^4$ = H or $CH_3$
$R^5$ = H or $C_1$—$C_4$ alkyl
$R^6$ = H
$R^7$ = H or $C_1$—$C_4$ alkyl
$R^8$ = H or $C_1$—$C_3$ alkyl and
X = H or $C_1$—$C_3$ alkyl,
for stabilising rubber or rubber latices.

**Revendication**

Utilisation de benzylphénols de formule générale I:

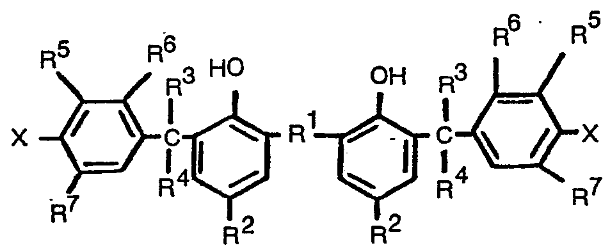 (I)

dans laquelle $R^1$ représente un des radicaux bivalents suivants:

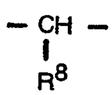 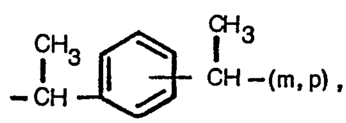 , 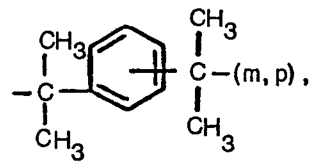 ,

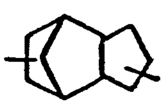 , 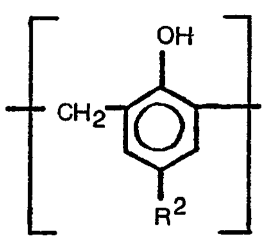 1-5, 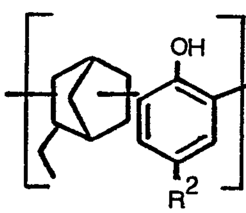 1-5, 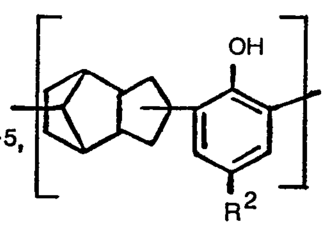 1-5,

$R^2$ = alkyle en $C_1$—$C_9$, benzyle, $\alpha$-méthylbenzyle, $\alpha,\alpha$-diméthylbenzyle,
$R^3$ = H, $CH_3$
$R^4$ = H, $CH_3$ ,

$R^5$ = H, alkyle en $C_1$—$C_4$
$R^6$ = H
$R^7$ = H, alkyle en $C_1$—$C_4$
$R^8$ = H, alkyle en $C_1$—$C_3$, et
X = H, alkyle en $C_1$—$C_3$,
en vue de stabiliser le caoutchouc ou les latex de caoutchouc.